# EUROPEAN PATENT SPECIFICATION

(11) **EP 0 370 497 B1**
(45) Date of publication and mention of the grant of the patent: **16.02.1994**
(21) Application number: 89121586.5
(22) Date of filing: 22.11.1989
(51) Int. Cl.: C07C 255/54, C09K 19/20, C07C 43/225, C07C 43/205, C07C 205/35, C07C 69/92, C07C 65/24

(54) **Optically active compound and ferroelectric liquid crystal composition containing the same**
Optisch aktive Verbindung und diese enthaltende flüssigkristalline ferroelektrische Zusammensetzung
Composé optiquement actif et composition de cristaux liquides ferro-électriques le contenant

(30) Priority: 24.11.1988 JP 296458/88
(43) Date of publication of application: 30.05.1990
(73) Proprietor: ARAKAWA KAGAKU KOGYO KABUSHIKI KAISHA, Osaka-shi, Osaka 541 (JP); SHARP CORPORATION, Osaka-shi (JP)
(72) Inventor: Sone, Takaaki, Amagasaki-shi Hyogo-ken (JP); Kajita, Kazushige, Osaka-shi (JP); Kuratate, Tomoaki, Nara-shi Nara-ken (JP); Kodey, Mitsuhiro, Nara-shi Nara-ken (JP); Funada, Fumiaki, Yamatokoriyama-shi Nara-ken (JP)
(74) Representative: Türk, Gille, Hrabal, Leifert

(56) References cited:
- EP-A- 0 251 335
- EP-A- 0 311 329
- US-A- 4 689 176
- J. AM. CHEM. SOC., vol. 108, 1986, pages 7067-7073, American Chemical Society; M.R. EDWARDS et al.: "Anatomy of an Sn1 reaction. crystal structure-reactivity correlations for 1-Arylethanol derivatives"
- J. AM. CHEM. SOC., vol. 106, pages 1361-1372, American Chemical Society; J.P. RICHARD et al.: "Formation and stability of ring-substituted 1-Phenylethyl carbocations"
- CHEMICAL ABSTRACTS, vol. 93, no. 15, 13th October 1980, page 695, abstract no. 150017v, Columbus, Ohio, US; & JP-A-79 125 652

## Description

The present invention relates to an optically active compound useful as an additive for forming a ferroelectric liquid crystal composition, a process for the preparation thereof and a ferroelectric liquid crystal composition containing the same.

The liquid crystal display cell or device most widely used at present is a twisted nematic (TN) mode display cell or device, but it has the defect that the speed of response is slow as compared with display systems of light emitting type such as electroluminescence display and plasma display. In order to improve the defect, various studies have been made.

For instance, a display system using ferroelectic liquid crystals is proposed as a liquid crystal display utilizing a different principle from the TN mode display by N A. Clark et al. in Applied Physics Letters, Vol. 36, 899(1980). This display system utilizes the chiral smectic C phase of ferroelectric liquid crystals. It is superior to the TN mode display in high speed responsibility and the like. The high speed responsibility would also make a large capacity display possible. Accordingly, as a ferroelectric liquid crystal display material there has been demanded a ferroelectric liquid crystal compound capable of exhibiting the chiral smectic C phase and having a large spontaneous polarization (Ps). However, no satisfactory ferroelectric liquid crystal has been provided.

It is known that a ferroelectic liquid crystal composition can be obtained by adding an optically active compound, which does not exhibit the mesophase in itself, to smectic liquid crystals. Since optically active compounds having structures which have hither to not attracted attention can be utilized in this composition, the scope of investigation for ferroelectric liquid crystal materials is further widened (cf. L. A. Bresner et al, Molecular Crystals and Liquid Crystals, Vol. 89, page 327, 1982). However, in general, it is difficult to obtain optically active compounds, excepting amino acids, organic acids and saccharides which are easily available by microbial fermentation or as natural products. In particular, there has not been accomplished a technique for producing optically active compounds suitable as an additive for forming ferroelectric liquid crystal compositions by adding to smectic liquid crystals.

Accordingly, it is a primary object of the present invention to provide an optically active compound having a high compatibility with known smectic liquid crystals and capable of producing a liquid crystal composition having a high Ps value when added to the smectic liquid crystals.

A further object of the present invention is to provide an optically active compound useful as an additive for nematic liquid crystals, which can induce a twisted structure in the molecular orientation of nematic liquid crystals when added to the nematic liquid crystals, and accordingly which is useful as an additive for preventing a so-called reverse domain of TN mode liquid crystal display cells or devices.

A still further object of the present invention is to provide a process for preparing the above-mentioned optically active compound.

Another object of the present invention is to provide a ferroelectric liquid crystal composition having a high speed responsiveness a wide working temperature range, an excellent orientation characteristic and an excellent memory effect.

A still another object of the present invention is to provide a nematic liquid crystal composition capable of providing TN mode liquid crystal display devices having no reverse domain.

These and other objects of the present invention will become apparent from the description hereinafter.

In accordance with the present invention, there is provided an optically active compound of the formula (1): 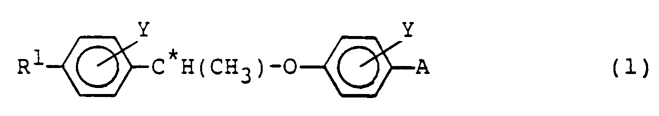
wherein R¹ is an alkyl or alkyloxy group having 1 to 18 carbon atoms, Y is a halogen atom, cyano group, nitro group, methyl group, hydrogen atom or trifluoromethyl group and two Y groups may be the same or different, and A is nitro group, cyano group, carboxyl group, hydrogen atom, a halogen atom, an alkyl group having 1 to 18 carbon atoms, an alkyloxy group having 1 to 18 carbon atoms, -CO-R² or 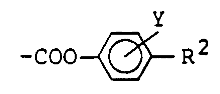
in which R² is an alkyl or alkyloxy group having 1 to 18 carbon atoms and Y is as defined above.

The optically active compound (1) can be easily prepared from a p-substituted α-methylbenzyl alcohol and a p-substituted or p-unsubstituted halobenzene or phenol compound by known etherification methods.

In another aspect of the present invention, there is provided a liquid crystal composition capable of exhibiting a chiral smectic C phase or a chiral nematic phase. The addition of the optically active compound (1) to smectic liquid crystals provides ferroelectric liquid crystal compositions. The addition of the optically active compound (1) to nematic liquid crystals provides liquid crystal compositions suitable for use in TN mode liquid crystal devices.

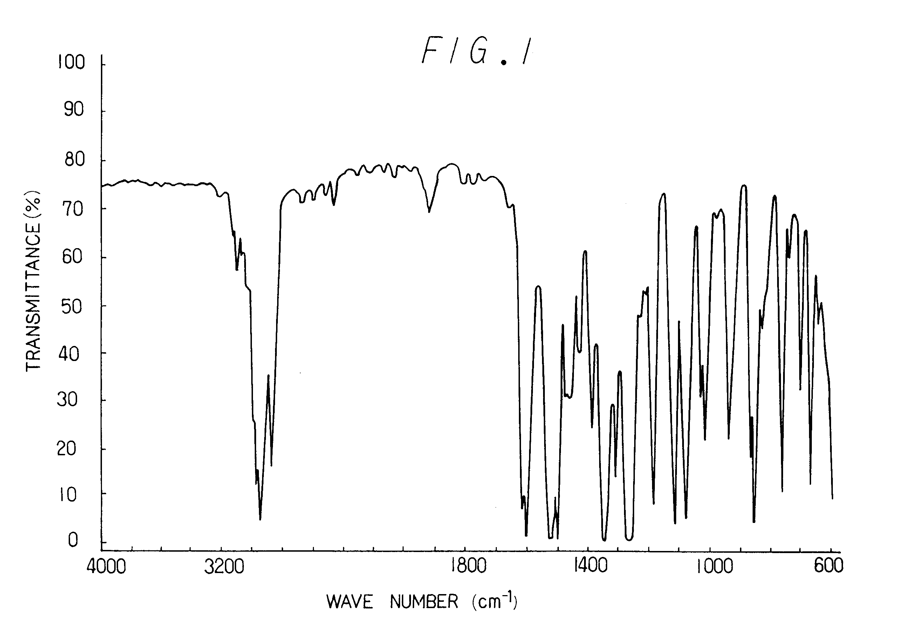

Figs. 1 to 5 are infrared absorption spectrums of representative optically active compounds of the present invention.

In the optically active compound of the present invention represented by the formula (1): 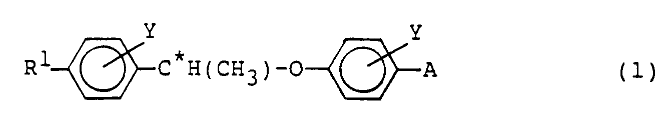
the group R¹ is an alkyl or alkyloxy group having 1 to 18 carbon atoms, preferably 5 to 12 carbon atoms. When the group R¹ is those having 19 or more carbon atoms, liquid crystal compositions obtained by adding the compound (1) to liquid crystals have a high viscosity and, therefore, the speed of response is low. Compounds having groups other than the alkyl and alkyloxy groups instead of the group R¹ defined above are not suitable, since they are apt to rapidly lower the thermal stability of the smectic phase when added to liquid crystals.

Examples of the alkyl group having 1 to 18 carbon atoms are, for instance, methyl, ethyl, propyl, isopropyl, butyl, isobutyl, pentyl, 1- or 2-methylbutyl, hexyl, 1- or 3-methylpentyl, heptyl, 1- or 4-methylhexyl, octyl, 1-methylheptyl, nonyl, 1- or 6-methyloctyl, decyl, 1-methylnonyl, undecyl, 1-methyldecyl, dodecyl, 1-methylundecyl groups, and the like. The alkyl group R¹ is not limited to the exemplified groups. The alkyl group R¹ may contain an asymmetric carbon atom.

Examples of the alkyloxy group having 1 to 18 carbon atoms are, for instance, methoxy, ethoxy, propoxy, isopropoxy, butoxy, isobutoxy, pentyloxy, 1- or 2-methylbutyloxy, hexyloxy, 1- or 3-methylpentyloxy, heptyloxy, 1- or 4-methylhexyloxy, octyloxy, 1-methylheptyloxy, nonyloxy, 1- or 6-methyloctyloxy, decyloxy, 1-methylnonyloxy, undecyloxy, 1-methyldecyloxy, dodecyloxy, 1-methylundecyloxy groups and the like. The alkyloxy group R¹ is not limited to the exemplified groups. The alkyloxy group R¹ may contain an asymmetric carbon atom.

The group A in the formula (1) is nitro group, cyano group, carboxyl group, hydrogen atom, a halogen atom, an alkyl or alkyloxy group having 1 to 18 carbon atoms, preferably 5 to 12 carbon atoms, a group of the formula: -CO-R² or a group of the formula: 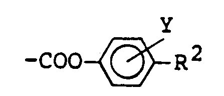
in which R² is an alkyl or alkyloxy group having 1 to 18 carbon atoms, preferably 5 to 12 carbon atoms.

A hydrogen atom in each of two benzene rings of the compound according to the present invention may be substituted by a halogen atom, cyano group, nitro group, methyl group or trifluoromethyl group, as defined by the group Y in the formula (1).

Representative examples of the optically active compound (1) are, for instance, 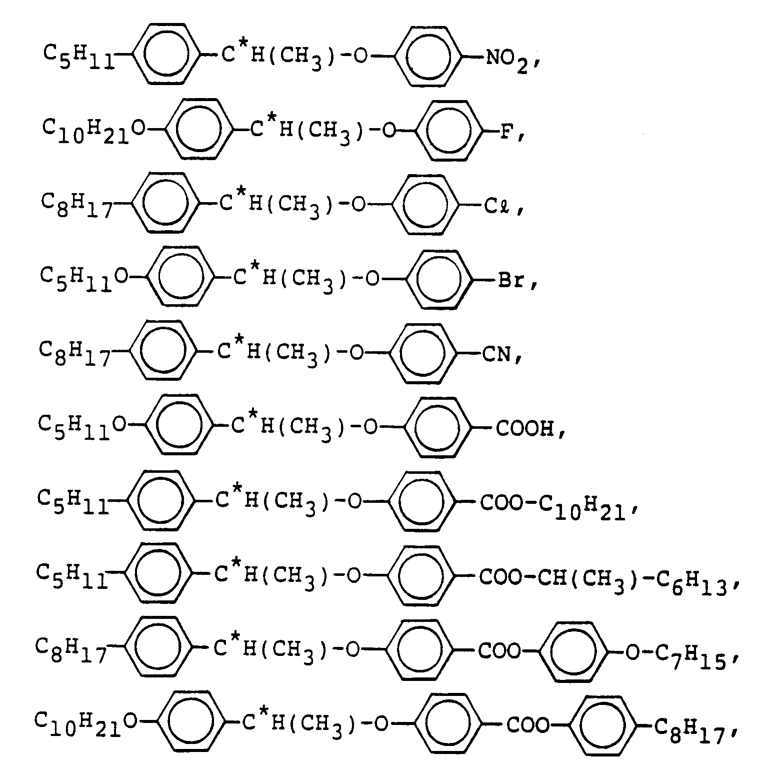
and the like.

The optically active compounds of the present invention may be either R-form or S-form. From the viewpoint of the response speed of the obtained liquid crystal compositions, it is preferable that the optical purity of the compound (1) is 100 %. However, if the optical purity is not less than about 95 %, the compounds (1) can be used without problems in ferroelectric liquid crystal display.

The optically active compounds (1) of the present invention are usually in such a state as liquid or crystalline state and are white or yellow, though it varies depending on the kinds of the groups R¹, A and Y.

The optically active compounds of the present invention have a high compatibility with many known liquid crystals and, therefore, they can be used in admixture therewith as a component of liquid crystal materials. In particular, when the compounds of the present invention are added to smectic liquid crystals, the obtained mixtures show chiral smectic C phase and can realize a high Ps value in the chiral smectic C phase and, therefore, the mixtures can be used in ferroelectric liquid crystal display.

The optically active compounds of the present invention are applicable to non-chiral smectic liquid crystal compounds, chiral smectic liquid crystal compounds, and blended smectic liquid crystals. Although the optically active compounds per se do not always show mesophase, the addition of an appropriate amount of the optically active compounds to the smectic liquid crystal materials causes or increases spontaneous polarization, thus providing ferroelectric liquid crystal compositions having a high response speed.

Also, since the optically active compounds of the present invention have an optically active asymmetric carbon, it is possible to induce a twisted structure in the molecular orientation of nematic liquid crystal, namely the cholesteric phase (chiral nematic phase), by adding the compounds of the invention to the nematic liquid crystals. Nematic liquid crystals having twisted molecular axis, namely chiral nematic liquid crystals, which show the cholesteric phase, do not cause a so-called reverse domain in the TN mode liquid crystal display. Accordingly, the optically active compounds of the invention are also useful as an additive for nematic liquid crystals, and the reverse domain in the TN mode liquid crystal display is prevented by the formation of nematic liquid crystal compositions capable of showing the cholesteric phase.

The optically active compounds (1) of the present invention can be prepared from an optically active p-substituted α-methylbenzyl alcohol by known etherification methods, for instance, by methods represented by the following reaction formulas (I) and (II). 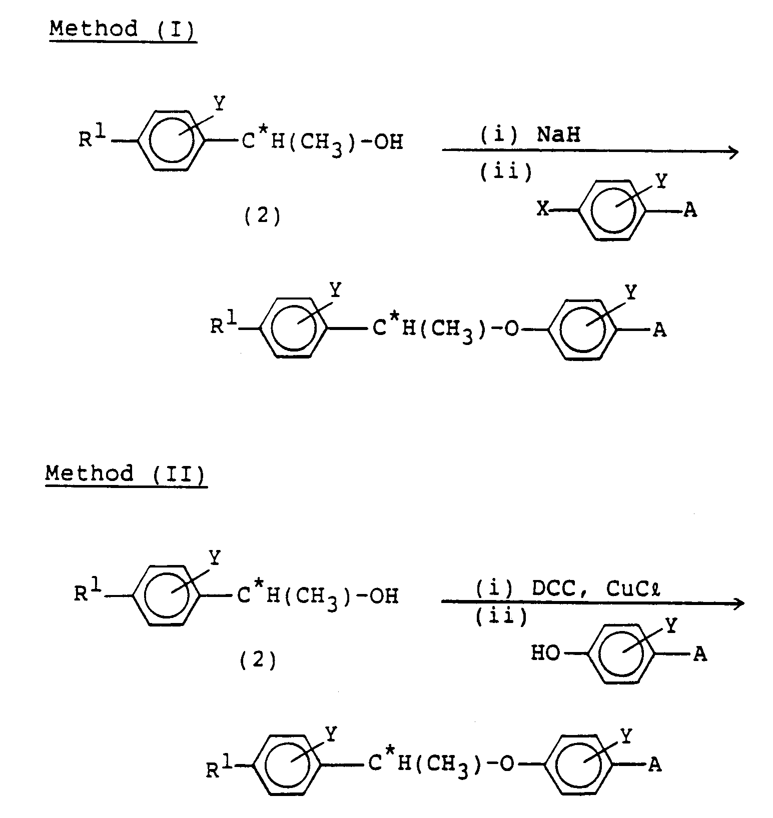
In the method (I), the etherification is carried out according to the Williamson's ether synthesis method. An optically active p-substituted α-methylbenzyl alcohol represented by the formula (2) wherein R¹ and Y are as defined above is converted into an alkoxide by a strong base, e.g. alkali metal hydride such as sodium hydride. The alkoxide is then reacted with a p-substituted or p-unsubstituted halobenzene compound of the formula (3): 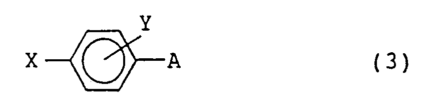
wherein X is a halogen atom, and Y and A are as defined above,
for example, p-substituted fluorobenzene compounds, p-substituted chlorobenzene compounds, fluorobenzene compounds and chlorobenzene compounds, whereby the optically active compounds (1) can be prepared in the state that the absolute configuration of the optically active alcohol (2) is kept.

In the method (II), the optically active alcohol (2) is reacted with dicyclohexylcarbodiimide (DCC) in a nitrogen atmosphere in the presence of cuprous chloride to produce an adduct (isourea) of the optically active alcohol (2) and DCC. The adduct is then reacted with a p-substituted or p-unsubstituted phenol compound (4): 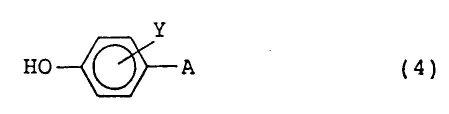
wherein A and Y are as defined above,
whereby the optically active compounds (1) can be prepared in the state that the absolute configuration of the optically active alcohol (2) is inverted. In the method (II), since the absolute configuration of the starting material is inverted by Walden inversion in the product, the S-form of the optically active alcohol (2) is used as the starting material when the R-form of the compound (1) is desired, and the R-form of the optically active alcohol (2) is used when the S-form of the compound (1) is desired.

The substituent groups at the para-position of the p-substituted halobenzene compounds in the method (I) and the p-substituted phenol compounds in the method (II) correspond to the group A of the final product (1). The substituent groups at the para-position are suitably selected in accordance with the desired products.

The optically active alcohols (2) used as the starting material in the present invention can be easily prepared by the method of optical resolution described in the copending Japanese Patent Application No. 63-38388. That is to say, the racemic mixture of the p-substituted α-methylbenzyl alcohol (2) is reacted with trichloroethyl butylate in an organic solvent such as ethyl ether, n-hexane or cyclohexane in the presence of an enzyme having an esterase activity at a temperature of 10° to 40°C, especially 25° to 30°C for 1 to 150 hours, especially 24 to 72 hours, whereby only the (+)-form of the compound (2) is converted into the butylate. After removing the esterase from the reaction mixture, and after further removing the organic solvent as occasion demands, the (-)-form of the compound (2) is separated from the resulting (+)-butylate, for instance, by silica gel column chromatography. The (+)-butylate can be converted into the (+)-form of p-substituted α-methylbenzyl alcohol (2) by the hydrolysis. The p-substituted α-methylbenzyl alcohol can be prepared, for instance, by reducing a p-substituted acetophenone with a reducing agent such as sodium borohydride in ethanol, or by reacting p-hydroxyacetophenone with an alkyl halide according to the Williamson's synthesis and reducing the resulting p-substituted acetophenone according to the above method.

The halobenzene compound (3) and the phenol compound (4) used in the above methods (I) and (II) may be those having the substituent group Y, i.e. nitro group, cyano group, a halogen atom, methyl group or trifluoromethyl group, at the ortho-position or meta-position of the benzene ring.

The optically active compounds of the present invention represented by the formula (1) wherein the group A is carboxyl group, can be prepared, for instance, by the following method (III) or (IV) wherein a cyano compound obtained by the method (I) or (II) [namely the compound (1) in which A is cyano group] or an ester compound obtained by the method (I) or (II) [namely the compound (1) in which A is -CO-R⁷ in which R⁷ is an alkyloxy group] is subjected to alkali hydrolysis. 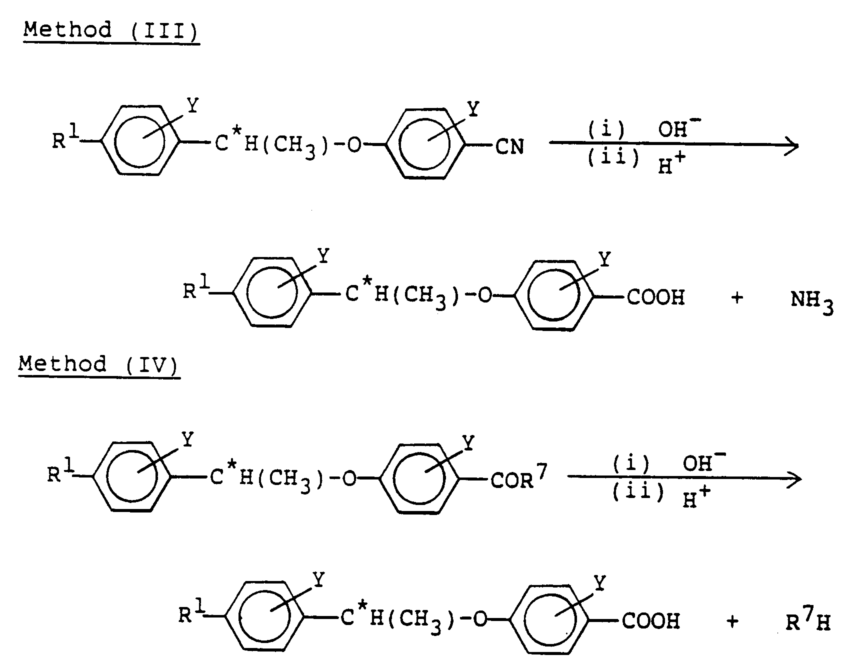
The optically active compound of the present invention represented by the formula (1) wherein the group A is 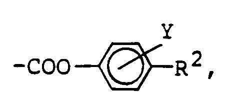
can be prepared, for instance, by the following method (V) wherein a carboxylic acid compound, namely the compound (1) in which A is carboxyl group, is esterified with a p-alkyl- or p-alkyloxy-phenol compound in the presence of DCC and 4-dimethylaminopyridine (DMAP). 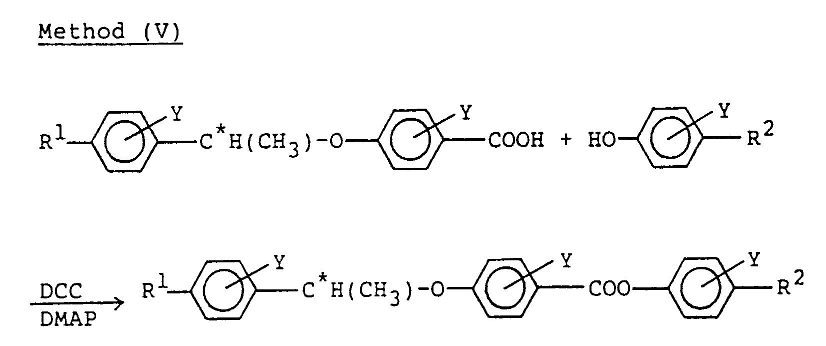
One or more kinds of the optically active compounds (1) of the present invention are admixed with known liquid crystal compounds or liquid crystal compositions, e.g. compounds or compositions capable of exhibiting the smectic C phase, and nematic liquid crystals or nematic liquid crystal compositions, to prepare the liquid crystal compositions of the present invention.

Examples of the compound capable of showing the smectic C phase to be admixed with the compounds (1) are, for instance, compounds represented by the formulas (5), (6) and (7): 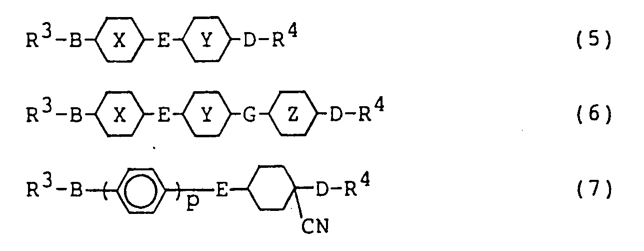
wherein each of B and D is single bond, -COO-, -OCO-, -CH=CH-COO-, -OCO-CH=CH-, -O-, -S-, -OCOO- or -CO-; each of E and G is single bond, -COO-, -OCO-, -CH=N-, -N=CH-, -CH=CH-, -C≡C-, -CH=CH-COO-, -OCO-CH=CH-, -CH₂CH₂-, -OCH₂-, -CH₂O-, -COS- or -SCO-; each of 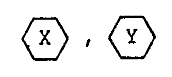
and 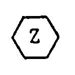
is a 6-membered ring such as benzene ring, cyclohexane ring, bicyclo[2,2,2]octane ring, pyridine ring, pyrimidine ring, pyrazine ring, pyridazine ring, piperazine ring, pyran ring, dioxacyclohexane ring, thiapyran ring, dithian ring, thiadiazine ring or tetrazine ring, or the 6-membered ring substituted by fluorine atom, chlorine atom, bromine atom, cyano group, nitro group, a lower alkyl group, a lower alkoxyl group or deuterium; each of R³ and R⁴ is a linear or branched alkyl group having 1 to 15 carbon atoms which may contain an asymmetric carbon atom; and p is 1 or 2.

Examples of the nematic liquid crystal are, for instance, compounds represented by the formulas (8), (9) and (10): 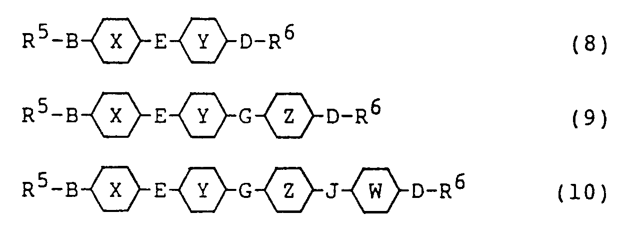
wherein B, D, E, G, 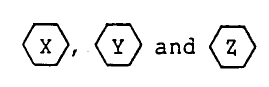
are as defined above, J is one of the groups defined above with respect to E and G, 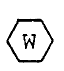
is one of the groups defined above with respect to 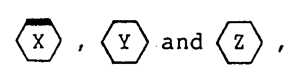
and each of R⁵ and R⁶ is cyano group, nitro group, CF₃ or a linear or branched alkyl group having 1 to 15 carbon atoms which may contain an asymmetric carbon atom.

The content of the optically active compound (1) in the liquid crystal composition according to the present invention is from 0.1 to 20 % by weight, preferably 0.5 to 10 % by weight. When the content of the compound (1) is less than 0.1 % by weight, the speed of response is low, and when the content of the compound (1) is more than 20 % by wegiht, the temperature range exhibiting the smectic C phase is apt to be lowered below the practical temperature range.

The liquid crystal composition of the present invention may contain known additives such as CT complex, ion dopant, surface active agent and activated alumina.

The liquid crystal composition containing at least one of the optically active compounds (1) according to the present invention prepared, for instance, by adding the compound (1) to a suitably selected liquid crystal compound or liquid crystal composition showing the smectic C phase, provides a ferroelectric liquid crystal composition having a large Ps, a high speed responsibility, an orientation characteristic having IAC or INAC phase series, and a good memory effect, and it can be effectively used in ferroelectric liquid crystal display.

The present invention is more specifically described and explained by means of the following Examples in which all % are by weight unless otherwise noted.

### Example 1

### (+)-4-n-Pentyl-α-methylbenzyl-4′-nitrophenyl ether [compound of the formula (1) wherein R¹ is C₅H₁₁ and A is NO₂]

Into 40 ml of anhydrous tetrahydrofuran was dispersed 3.20 g of sodium hydride (purity: 60 %, 80 millimoles), and 9.62 g (50 millimoles) of (+)-4-n-pentyl-α-methylbenzyl alcohol having a specific rotation [α]$\frac{\text{20}}{\text{D}}$ of +39.7° (c=1.00, chloroform) was added dropwise to the resulting suspension with cooling with ice. The mixture was further agitated for 1 hour with cooling with ice. To the mixture was then added dropwise an anhydrous tetrahydrofuran solution of 8.47 g (60 millimoles) of p-fluoronitrobenzene with cooling with ice, and the reaction was further conducted at room temperature for 12 hours with stirring.

Water was carefully added to the reaction mixture to decompose excess sodium hydride, and 150 mℓ of diethyl ether was added to the reaction mixture to extract the product. The ether extract was washed with 2N aqueous solution of sodium hydroxide 2 times and then with water 3 times. The resulting ether layer was dried with anhydrous magnesium sulfate and ether was removed to give 14.47 g of crude product. The crude product was purified by silica gel chromatography (n-hexane/ethyl acetate = 30/1 by volume) to give 10.96 g of the desired product in the state of a yellow liquid. The yield was 70 %, and the specific rotation [α]$\frac{\text{25}}{\text{D}}$ was +85.1° (c=1.13, chloroform).

The infrared absorption spectrum of the product is shown in Fig. 1.

The δ values (p.p.m.) of ¹H-NMR spectrum of the product (300 MH_{z}, CDCℓ₃, TMS internal standard) were as follows:
8.08 (d, J=9Hz, 2H, benzene ring)
7.24 (d, J=8Hz, 2H, benzene ring)
7.16 (d, J=8Hz, 2H, benzene ring)
6.90 (d, J=9Hz, 2H, benzene ring)
5.37 (q, J=6Hz, 1H, -C*H(CH₃)-O-)
2.57 (t, J=8Hz, 2H, 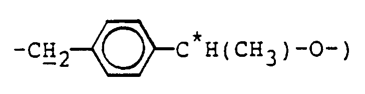
1.67 (d, J=6Hz, 3H, -C*H(CH₃)-O-)
1.5-1.6 (m, 2H, 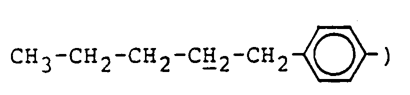
1.25-1.40 (m, 4H, 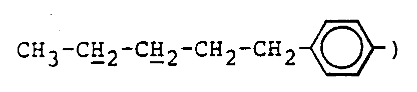
0.88 (t, J=7Hz, 3H, CH₃-CH₂-)
From the above analytical data, it was confirmed that the product was (+)-4-n-pentyl-α-methylbenzyl-4′-nitrophenyl ether.

### Example 2

### (-)-4-n-Octyl-α-methylbenzyl-4′-cyanophenyl ether [compound of the formula (1) wherein R¹ is C₈H₁₇ and A is CN]

Into 40 ml of anhydrous tetrahydrofuran was dispersed 3.20 g of sodium hydride (purity: 60 %, 80 millimoles), and 11.72 g (50 millimoles) of (-)-4-n-octyl-α-methylbenzyl alcohol having a specific rotation [α]$\frac{\text{25}}{\text{D}}$ of -32.0° (c=1.60, chloroform) was added dropwise to the resulting suspension with cooling with ice. The mixture was further agitated for 1 hour with cooling with ice. To the mixture was then added dropwise an anhydrous tetrahydrofuran solution of 7.27 g (60 millimoles) of p-fluorobenzonitirile with cooling with ice, and the reaction was further conducted at room temperature for 12 hours with stirring.

Water was carefully added to the reaction mixture to decompose excess sodium hydride, and 300 mℓ of diethyl ether was added to the reaction mixture to extract the product. The ether extract was washed with 2N aqueous solution of sodium hydroxide 2 times and then with water 3 times. The resulting ether layer was dried with anhydrous magnesium sulfate and ether was removed to give 18.36 g of crude product. The crude product was purified by silica gel chromatography (n-hexane/ethyl acetate = 50/1 by volume) to give 13.47 g of the desired product in the state of a light yellow liquid. The yield was 80 %, and the specific rotation [α]$\frac{\text{25}}{\text{D}}$ was -35.4° (c=1.09, chloroform).

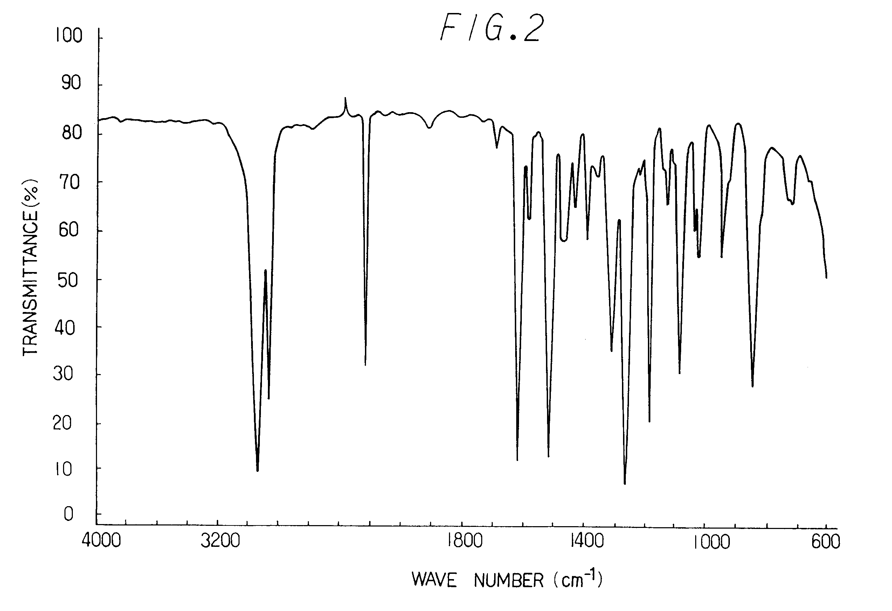

The infrared absorption spectrum of the product is shown in Fig. 2.

The δ values (p.p.m.) of ¹H-NMR spectrum of the product (300 MH_{z}, CDCℓ₃, TMS internal standard) were as follows:
7.46 (d, J=9Hz, 2H, benzene ring)
7.23 (d, J=8Hz, 2H, benzene ring)
7.15 (d, J=8Hz, 2H, benzene ring)
6.88 (d, J=9Hz, 2H, benzene ring)
5.32 (q, J=6Hz, 1H, -C*H(CH₃)-O-)
2.56 (t, J=8Hz, 2H, 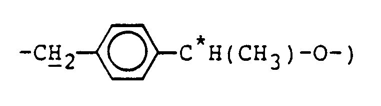
1.64 (d, J=6Hz, 3H, -C*H(CH₃)-O-)
1.5-1.6 (m, 2H, 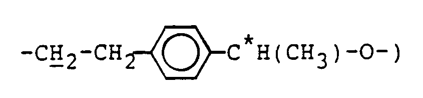
1.2-1.4 (m, 10H, 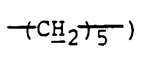
0.87 (t, J=7Hz, 3H, 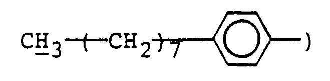
From the above analytical data, it was confirmed that the product was (-)-4-n-octyl-α-methylbenzyl-4′-cyanophenyl ether.

### Example 3

### (-)-4-(4′-n-Octyl-α-methylbenzyloxy)benzoic acid [compound of the formula (1) wherein R¹ is C₈H₁₇ and A is COOH]

(-)-4-n-Octyl-α-methylbenzyl-4′-cyanophenyl ether was prepared in the same manner as in Example 2, and the alkali hydrolysis was conducted by refluxing 9.00 g (27 millimoles) of the obtained ether for 24 hours in a solution of 11.2 g (280 millimoles) of sodium hydroxide in a mixed solvent of 20 mℓ of ethanol and 50 mℓ of water. The reaction mixture was acidified with hydrochloric acid, and the resulting white crystals were filtered off, washed with water and dried to give 8.36 g of crude product. The crude product was recrystallized from ethyl alcohol to give 5.86 g of the desired product (yield 62 %). The product had a melting point of 99°C and a specific rotation [D]$\frac{\text{25}}{\text{D}}$ of -19.2° (c=1.03, acetic acid).

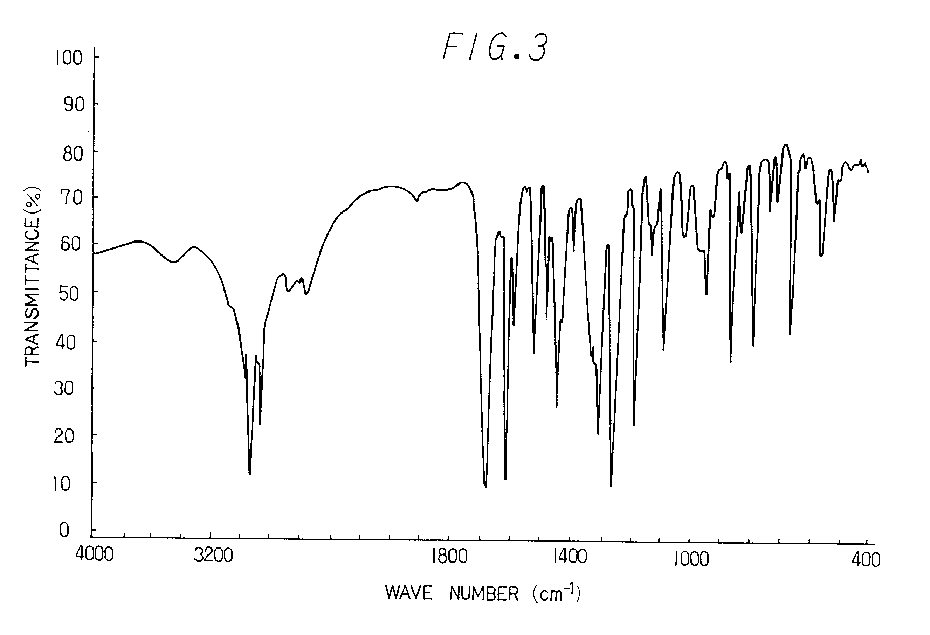

The infrared absorption spectrum of the product is shown in Fig. 3.

The δ values (p.p.m.) of ¹H-NMR spectrum of the product (300 MHz, DMSO-d6) were as follows:
12.58 (s, 1H, -COOH)
7.79 (d, J=9Hz, 2H, benzene ring)
7.29 (d, J=8Hz, 2H, benzene ring)
7.13 (d, J=8Hz, 2H, benzene ring)
6.95 (d, J=9Hz, 2H, benzene ring)
5.55 (q, J=6Hz, 1H, -C*H(CH₃)-O-)
2.49 (t, J=7Hz, 2H, 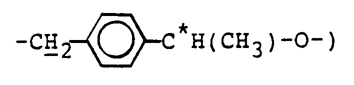
1.53 (d, J=6Hz, 3H, -C*H(CH₃)-O)
1.4-1.6 (m, 2H, 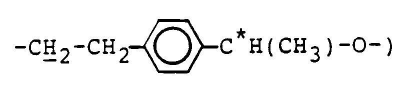
1.2-1.3 (m, 10H, 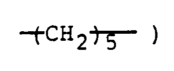
0.87 (t, J=7Hz, 3H, 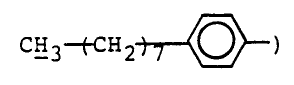
From the above analytical data, it was confirmed that the product was (-)-4-(4′-n-octyl-α-methylbenzyloxy)benzoic acid.

### Example 4

### (-)-4-(4′-n-Octyl-α-methylbenzyloxy)benzoic acid 4˝-n-heptyloxyphenyl ester [comound of the formula (1) wherein R¹ is C₈H₁₇ and A is

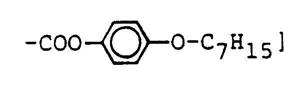

(-)-4-(4′-n-Octyl-α-methylbenzyloxy)benzoic acid was prepared in the same manner as in Example 3, and 1.06 g (3.0 millimoles) thereof was dissolved in 40 mℓ of dichloromethane together with 0.68 g (3.3 millimoles) of dicyclohexylcarbodiimide, 0.12 g (1.0 millimole) of dimethylaminopyridine and 0.63 g (3.0 millimoles) of p-n-heptyloxyphenol. The reaction was conducted at room temperature for 24 hours with stirring. The resulting crystals were filtered off. The dichloromethane filtrate was washed with hydrochloric acid 2 times and then with water 3 times and was dried with anhydrous magnesium sulfate. Dichloromethane was removed to give 1.82 g of crude product, and it was purified by silica gel chromatography (n-hexane/benzene = 3/2 by volume) to give 1.18 g of the desired product in the state of white crystals (yield 72 %). The product had a melting point of 49° to 51°C and a specific rotation [α]$\frac{\text{25}}{\text{D}}$ of -35.4° (c=1.01, chloroform).

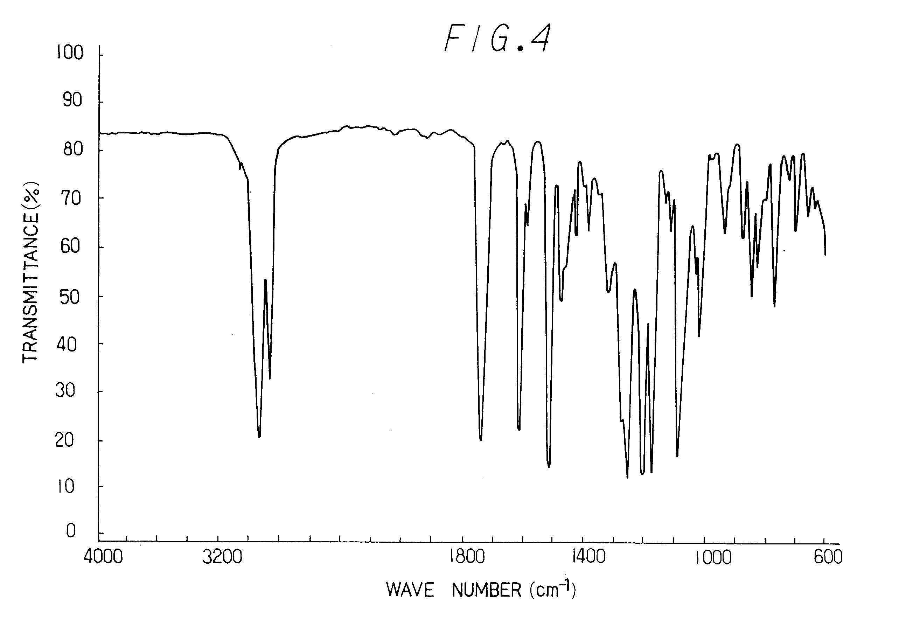

The infrared absorption spectrum of the product is shown in Fig. 4.

The δ values (p.p.m.) of ¹H-NMR spectrum of the product (300 MHz, CDCℓ₃, TMS internal standard) were as follows:
8.04 (d, J=9Hz, 2H, benzene ring)
7.26 (d, J=8Hz, 2H, benzene ring)
7.15 (d, J=8Hz, 2H, benzene ring)
7.05 (d, J=9Hz, 2H, benzene ring)
6.92 (d, J=9Hz, 2H, benzene ring)
6.89 (d, J=9Hz, 2H, benzene ring)
5.39 (q, J=6Hz, 1H, -C*H(CH₃)-O-)
3.96 (t, J=7Hz, 2H, 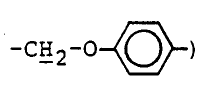
2.57 (t, J=8Hz, 2H, 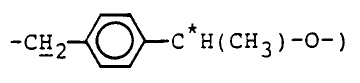
1.7-1.8 (m, 2H, -CH₂-)
1.66 (d, J=6Hz, 3H, -C*H(CH₃)-O-)
1.5-1.6 (m, 2H, -CH₂-)
1.2-1.5 (m, 18H, 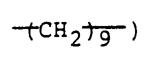
0.89 (t, J=7Hz, 3H, CH₃-)
0.87 (t, J=7Hz, 3H, CH₃-)
From the above analytical data, it was confirmed that the product was (-)-4-(4′-n-octyl-α-methylbenzyloxy)benzoic acid 4˝-n-heptyloxyphenyl ester.

### Example 5

### (+)-4-n-Pentyl-α-methylbenzyl-4′-n-heptylphenylether [compound of the formula (1) wherein R¹ is C₅H₁₁ and A is C₇H₁₅]

Three grams (16 millimoles) of (-)-4-pentyl-α-methylbenzyl alcohol having a specific rotation [α]$\frac{\text{25}}{\text{D}}$ of -38.2° (c=1.00, chloroform) was admixed with 3.20 g (16 millimoles) of dicyclohexylcarbodiimide and 30 mg (0.3 millimole) of cuprous chloride, and the reaction was carried out in a nitrogen atmosphere at room temperature for 24 hours with stirring. To the reaction mixture was added dropwise a solution of 2.50 g (13 millimoles) of p-n-heptylphenol dissolved in 20 mℓ of dichloromethane, and the reaction was carried out in a nitrogen atmosphere at room temperature for 24 hours with stirring. The resulting precipitate was removed by filtration, and the filtrate was diluted with 50 mℓ of diethyl ether, washed with a saturated aqueous solution of sodium hydrogencarbonate 2 times and then with water 3 times and dried with anhydrous magnesium sulfate. The solvent was removed to give 5.59 g of crude product, and it was purified by silica gel chromatography (n-hexane/ethyl acetate= 19/1 by volume) to give 2.35 g of desired product in the state of light yellow liquid (yield 49 %). The product had a specific rotation [α]$\frac{\text{25}}{\text{D}}$ was +27.3° (c=1.42, chloroform).

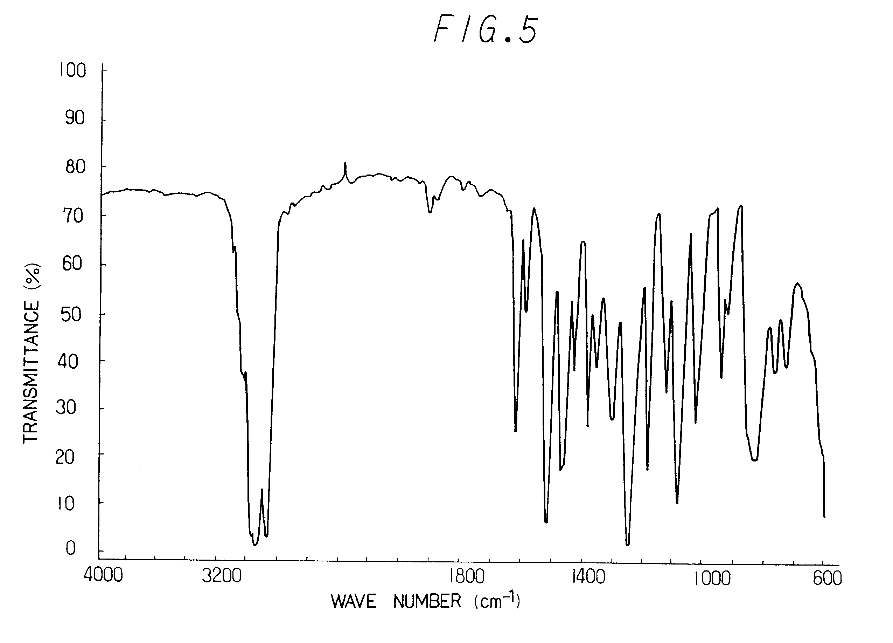

The infrared absorption spectrum of the product is shown in Fig. 5.

The δ values (p.p.m.) of ¹H-NMR spectrum of the product (300 MHz, CDCl₃, TMS internal standard) were as follows:
7.28 (d, J=8Hz, 2H, benzene ring)
7.14 (d, J=8Hz, 2H, benzene ring)
7.00 (d, J=9Hz, 2H, benzene ring)
6.78 (d, J=9Hz, 2H, benzene ring)
5.24 (q, J=6Hz, 1H, -C*H(CH₃)-O-)
2.86 (t, J=8Hz, 2H, 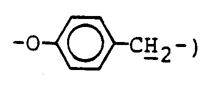
2.57 (d, J=8Hz, 2H, 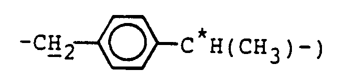
1.61 (d, J=6Hz, 3H, -C*H(CH₃)-O-)
1.5-1.7 (m, 4H, 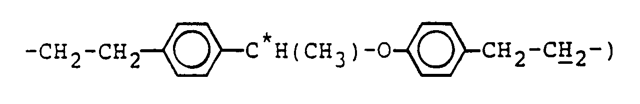
1.2-1.4 (m, 12H, 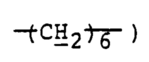
0.89 (t, J=6Hz, 3H, CH₃(̵CH₂)̵)
0.87 (t, J=7Hz, 3H, CH₃(̵CH₂)̵)
From the above analytical data, it was confirmed that the product was (+)-4-n-pentyl-α-methylbenzyl-4′-n-heptylphenylether.

### Example 6

A ferroelectric liquid crystal composition was prepared by using the optically active compound represented by the formula (1) and was applied to a ferroelectric liquid crystal display device as follows:

Firstly, a non-chiral liquid crystal composition (A) composed of the following ingredients was prepared.

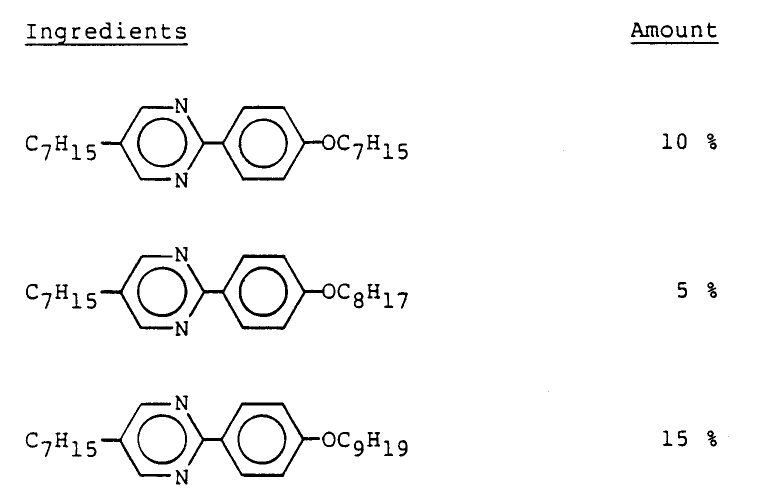

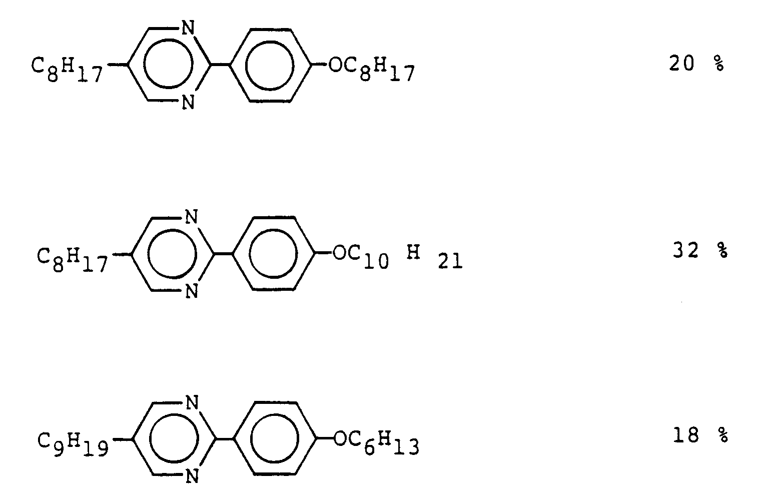

The liquid crystal composition (A) shows the smectic C phase at room temperature, and the phase change thereof is as follows: 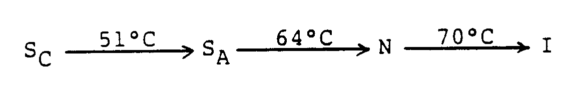
- S_{C}:: smectic C phase
- S_{A}:: smectic A phase
- N:: nematic phase
- I:: isotropic liquid phase
The optically active compound of the formula: 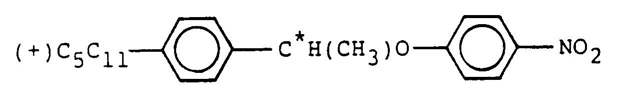
prepared in Example 1 was added to the liquid crystal composition (A) in an amount of 2 % to give a liquid crystal composition (B).

The liquid crystal composition (B) showed the following phase change. 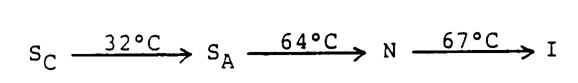
An ITO (indium tin oxide) film was formed on each of two glass substrates, and thereon was formed a SiO₂ layer. A polyamide film was formed on each SiO₂ layer by coating and was then rubbed. The two glass substrates were mounted so that the coated surfaces are facing at a spacing of 2 µm. The liquid crystal composition (B) was then introduced between the glass substrates. The thus prepared cell was heated once to 75°C in order to change the liquid crystal composition to the isotropic liquid, and cooled at a rate of 1°C/minute to room temperature, whereby a good orientation was obtained.

The liquid crystal display cell was placed between two crossed polarizers, and rectangular pulse of Vₚ₋ₚ=20V was applied, whereby change in intensity of transmitted light was observed. The speed of response obtained from the change in transmitted light intensity was 340 µsec. at 25°C.

### Example 7

The optically active compound represented by the formula: 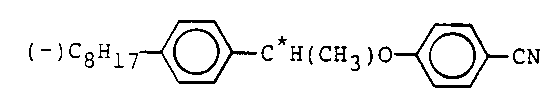
obtained in Example 2 was added to the non-chiral liquid crystal composition (A) in an amount of 2 % in the same manner as in Example 6, to give a liquid crystal composition (C).

The liquid crystal composition (C) showed the following phase change. 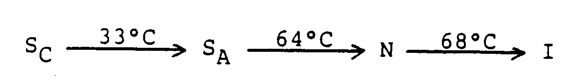
A liquid crystal cell was assembled in the same manner as in Example 6, using the liquid crystal composition (C). The speed of response obtained from the change in transmitted light intensity by application of rectangular pulse of Vₚ₋ₚ=20V to the cell was 370 µsec. at 25°C.

### Example 8

The optically active compound represented by the formula: 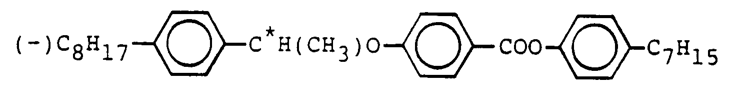
obtained in Example 4 was added to the non-chiral liquid crystal composition (A) in an amount of 2 % to give a liquid crystal composition (D).

The liquid crystal composition (D) showed the following phase change. 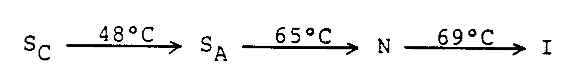
A liquid crystal cell was assembled in the same manner as in Example 6, using the liquid crystal composition (D). The speed of response obtained from the change in transmitted light intensity by application of rectangular pulse of Vₚ₋ₚ=20V to the cell was 320 µsec. at 25°C.

### Example 9

A nematic liquid crystal composition (nematic liquid crystal E8 made by BDH Limited.), which shows a phase change at -12°C from crystal phase to nematic phase and at 71°C from nematic phase to isotropic liquid phase and has a birefringence of 0.247, was introduced into a TN mode liquid crystal display cell, and was observed by a polarization microscope. Occurrence of reverse twisted domain was observed.

To the nematic liquid crystal composition was added 0.1 % of the optically active compound represented by the formula: 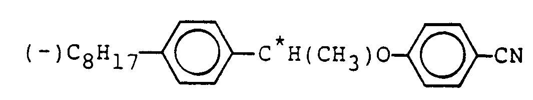
obtained in Example 2 to give a liquid crystal composition (E).

The liquid crystal composition (E) was introduced intc the TN mode liquid crystal display cell and was observed by a polarization microscope. The composition (E) showed no reverse twisted domain, and a homogeneous nematic phase was observed.

In addition to the ingredients used in the Examples, other ingredients can be used in the Examples as set forth in the specification to obtain substantially the same results.

## Claims

1. An optically active compound of the formula (1): 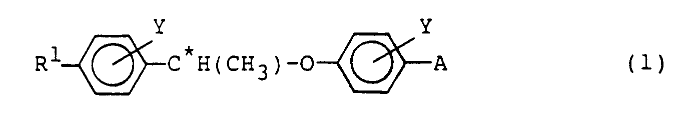 wherein R¹ is an alkyl or alkyloxy group having 1 to 18 carbon atoms, Y is a halogen atom, cyano group, nitro group, methyl group, hydrogen atom or trifluoromethyl group and two Y groups may be the same or different, and A is nitro group, cyano group, carboxyl group, hydrogen atom, a halogen atom, an alkyl group having 1 to 18 carbon atoms, an alkyloxy group having 1 to 18 carbon atoms, -CO-R² or 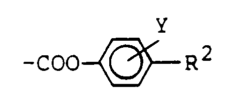 in which R² is an alkyl or alkyloxy group having 1 to 18 carbon atoms and Y is as defined above.

2. A process for preparing an optically active compound of the formula (1): 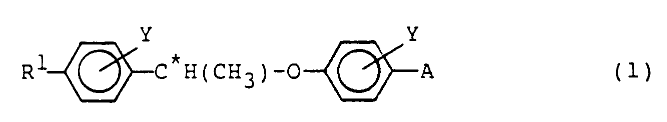 wherein R¹ is an alkyl or alkyloxy group having 1 to 18 carbon atoms, Y is a halogen atom, cyano group, nitro group, methyl group, hydrogen atom or trifluoromethyl group and two Y groups may be the same or different, and A is nitro group, cyano group, carboxyl group, hydrogen atom, a halogen atom, an alkyl group having 1 to 18 carbon atoms, an alkyloxy group having 1 to 18 carbon atoms, -CO-R² or 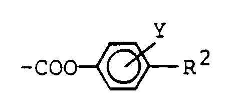 in which R² is an alkyl or alkyloxy group having 1 to 18 carbon atoms and Y is as defined above, which comprises subjecting an optically active p-substituted α-methybenzyl alcohol of the formula (2): 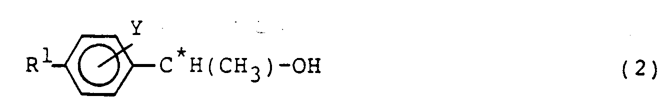 wherein R¹ and Y are as defined above, to etherification with a compound of the formula: 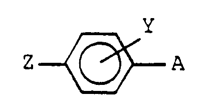 wherein A and Y are as defined above, and Z is a halogen atom or hydroxyl group.

3. The process of Claim 2, wherein said etherification is carried out by reacting the alkoxide of the optically active alcohol (2) with a halogenated benzene compound of the formula (3): 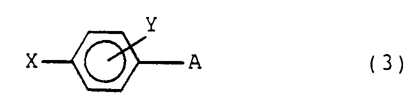 wherein X is a halogen atom, and A and Y are as defined above,
according to the Williamson's ether synthesis.

4. The process of Claim 2, wherein said etherification is carried out by reacting the optically active alcohol (2) with dicyclohexylcarbodiimide in the presence of cuprous chloride in a nitrogen atmosphere, and reacting the resulting adduct with a phenol compound of the formula (4): 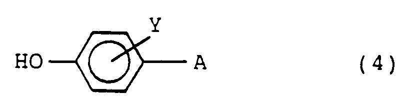 wherein A and Y are as defined above.

5. A liquid crystal composition comprising a liquid crystal material and an optically active compound of the formula (1): 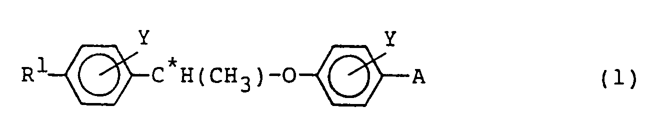 wherein R¹ is an alkyl or alkyloxy group having 1 to 18 carbon atoms, Y is a halogen atom, cyano group, nitro group, methyl group, hydrogen atom or trifluoromethyl group and two Y groups may be the same or different, and A is nitro group, cyano groups carboxyl group, hydrogen atom, a halogen atom, an alkyl group having 1 to 18 carbon atoms, an alkyloxy group having 1 to 18 carbon atoms, -CO-R² or 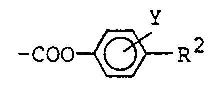 in which R² is an alkyl or alkyloxy group having 1 to 18 carbon atoms and Y is as defined above.

6. The composition of Claim 5, wherein the content of said optically active compound (1) is from 0.1 to 20 % by weight.

7. The composition of Claim 5, which shows the chiral smectic C phase.

8. The composition of Claim 5, which shows the chiral nematic phase.

9. Use of the optically active compound of Claim 1 as an additive for liquid crystals.

## Patentansprüche

1. Optisch aktive Verbindung der Formel (1): 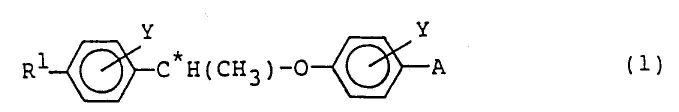 worin bedeuten:
R¹ eine Alkyl- oder Alkyloxygruppe mit 1 bis 18 Kohlenstoffatomen,
Y ein Halogenatom, eine Cyanogruppe, eine Nitrogruppe, eine Methylgruppe, ein Wasserstoffatom oder eine Trifluoromethylgruppe, wobei zwei Y-Gruppen gleich oder verschieden sein können, und
A eine Nitrogruppe, eine Cyanogruppe, eine Carboxylgruppe, ein Wasserstoffatom, ein Halogenatom, eine Alkylgruppe mit 1 bis 18 Kohlenstoffatomen, eine Alkyloxygruppe mit 1 bis 18 Kohlenstoffatomen, -CO-R² oder 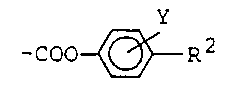 worin R² für eine Alkyl- oder Alkyloxygruppe mit 1 bis 18 Kohlenstoffatomen steht und Y wie oben definiert ist.

2. Verfahren zur Herstellung einer optisch aktiven Verbindung der Formel (1): 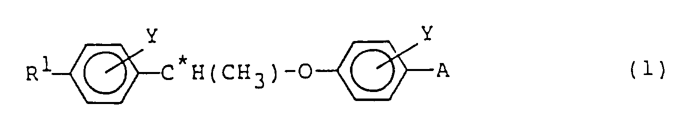 worin bedeuten:
R¹ eine Alkyl- oder Alkyloxygruppe mit 1 bis 18 Kohlenstoffatomen,
Y ein Halogenatom, eine Cyanogruppe, eine Nitrogruppe, eine Methylgruppe, ein Wasserstoffatom oder eine Trifluoromethylgruppe und worin zwei Y-Gruppen gleich oder verschieden sein können, und
A eine Nitrogruppe, eine Cyanogruppe, eine Carboxylgruppe, ein Wasserstoffatom, ein Halogenatom, eine Alkylgruppe mit 1 bis 18 Kohlenstoffatomen, eine Alkyloxygruppe mit 1 bis 18 Kohlenstoffatomen, -CO-R² oder 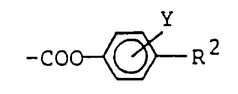 worin R² für eine Alkyl- oder Alkyloxygruppe mit 1 bis 18 Kohlenstoffatomen steht und Y wie oben definiert ist,
das umfaßt das Veräthern eines optisch aktiven p-substituierten α-Methylbenzylalkohols der Formel (2) 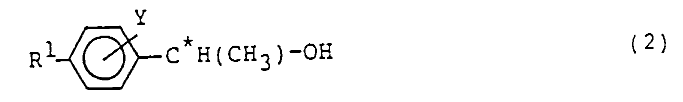 worin R¹ und Y wie oben definiert sind,
mit einer Verbindung der Formel 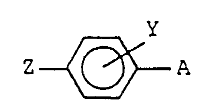 worin A und Y wie oben definiert sind und Z ein Halogenatom oder eine Hydroxylgruppe bedeutet.

3. Verfahren nach Anspruch 2, worin die Verätherung durchgeführt wird durch Umsetzung des Alkylats des optisch aktiven Alkohols (2) mit einer halogenierten Benzol-Verbindung der Formel (3): 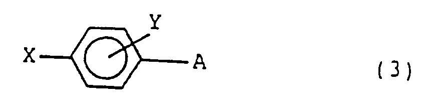 worin X für ein Halogenatom steht und A und Y wie oben definiert sind,
nach der Williamson-Äthersynthese.

4. Verfahren nach Anspruch 2, worin die Verätherung durchgeführt wird durch Umsetzung des optisch aktiven Alkohols (2) mit Dicyclohexylcarbodiimid in Gegenwart von Kupfer(I)chlorid in einer Stickstoffatomosphäre und Umsetzung des resultierenden Addukts mit einer Phenol-Verbindung der Formel (4) 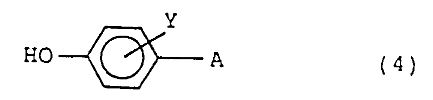 worin A und Y wie oben definiert sind.

5. Flüssigkristall-Zusammensetzung, die umfaßt ein Flüssigkristall-Material und eine optisch aktive Verbindung der Formel (1) 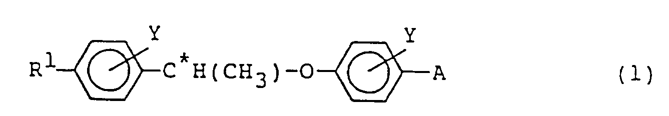 worin bedeuten:
R¹ eine Alkyl- oder Alkyloxygruppe mit 1 bis 18 Kohlenstoffatomen,
Y ein Halogenatom, eine Cyanogruppe, eine Nitrogruppe, eine Methylgruppe, ein Wasserstoffatom oder eine Trifluoromethylgruppe, wobei zwei Y-Gruppen gleich oder verschieden sein können, und
A eine Nitrogruppe, eine Cyanogruppe, eine Carboxylgruppe, ein Wasserstoffatom, ein Halogenatom, eine Alkylgruppe mit 1 bis 18 Kohlenstoffatomen, eine Alkyloxygruppe mit 1 bis 18 Kohlenstoffatomen, -CO-R² oder 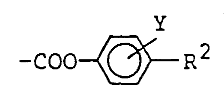 worin R² für eine Alkyl- oder Alkyloxygruppe mit 1 bis 18 Kohlenstoffatomen steht und Y wie oben definiert ist.

6. Zusammensetzung nach Anspruch 5, worin der Gehalt an der optisch aktiven Verbindung (1) 0,1 bis 20 Gew.-% beträgt.

7. Zusammensetzung nach Anspruch 5, die eine chirale smektische C-Phase aufweist.

8. Zusammensetzung nach Anspruch 5, die eine chirale nematische Phase aufweist.

9. Verwendung der optisch aktiven Verbindung nach Anspruch 1 als Zusatz für Flüssigkristalle.

## Revendications

1. Un composé optiquement actif ayant la formule (1) : 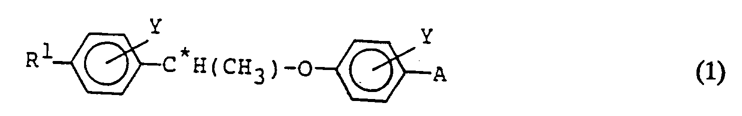 où R¹ est a groupe alkyle ou alkyloxy ayant 1 à 18 atomes de carbone, Y est un atome d'halogène, un groupe cyano, a groupe nitro, un groupe méthyle, un atome d'hydrogène ou un groupe trifluorométhyle et où deux groupes Y peuvent être identiques ou différents, et où A est un groupe nitro, un groupe cyano, un groupe carboxyle, a atome d'hydrogène, un atome d'halogène, un groupe alkyle ayant 1 à 18 atomes de carbone, un groupe alkyloxy ayant 1 à 18 atomes de carbone, -CO-R² ou 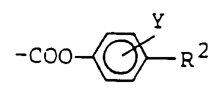 où R² est un groupe alkyle ou alkyloxy ayant 1 à 18 atomes de carbone et Y est tel que défini ci-dessus.

2. Un procédé de préparation d'un composé optiquement actif ayant la formule (1): 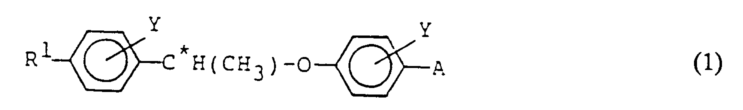 où R¹ est un groupe alkyle ou alkyloxy ayant 1 à 18 atomes de carbone, Y est un atome d'halogène, un groupe cyano, un groupe nitro, un groupe méthyle, un atome d'hydrogène ou un groupe trifluorométhyle et où deux groupes Y peuvent être identiques ou différents, et où A est un groupe azote, un groupe cyano, un groupe carboxyle, un atome d'hydrogène, un atome d'halogène, un groupe alkyle ayant 1 à 18 atomes de carbone, un groupe alkyloxy ayant 1 à 18 atomes de carbone, -CO-R² ou 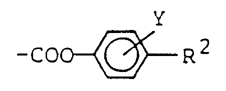 où R² est un groupe alkyle ou alkyloxy ayant 1 à 18 atomes de carbone et Y est tel que défini ci-dessus, comprenant le fait de soumettre un alcool α-méthylbenzylique substitué p optiquement actif ayant la formule (2): 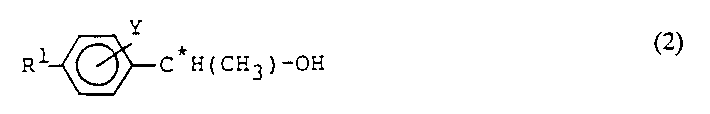 où R¹ et Y sont tels que définis ci-dessus, à une éthérification avec un composé ayant la formule : 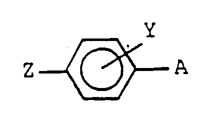 où A et Y sont tels que définis ci-dessus, et Z est un atome d'halogène ou un groupe hydroxyle.

3. Le procédé selon la Revendication 2, dans lequel ladite éthérification est effectuée en faisant réagir l'alkoxyde de alcool optiquement actif (2) avec un composé benzène halogéné ayant la formule (3): 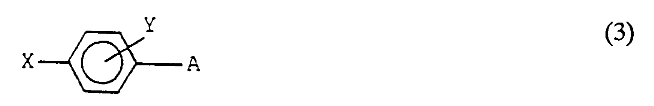 où X est un atome d'halogène, et A et Y sont tels que définis ci-dessus, conformément à la synthèse d'éther de Williamson.

4. Le procédé selon la Revendication 2, dans lequel ladite éthérification est effectuée en faisant réagir l'alcool optiquement actif (2) avec de la dicyclohexylcarbodiimide en présence de chlorure de cuivre sous atmosphère d'azote, et en faisant réagir l'additif qui en résulte avec un composé phénol ayant la formule (4): 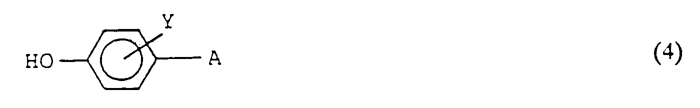 où A et Y sont tels que définis ci-dessus.

5. Une composition de cristaux liquides comprenant un matériau de cristaux liquides et un composé optiquement actif ayant la formule (1): 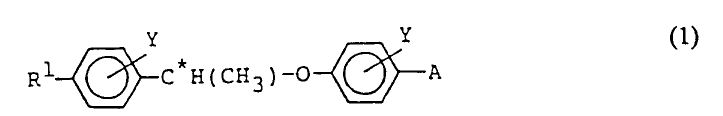 où R¹ est un groupe alkyle ou alkyloxy ayant 1 à 18 atomes de carbone, Y est un atome d'halogène, un groupe cyano, un groupe nitro, un groupe méthyle, un atome d'hydrogène ou un groupe trifluorométhyle et où deux groupes Y peuvent être identiques ou différents, et où A est un groupe nitro, un groupe cyano, un groupe carboxyle, un atome d'hydrogène, a atome d'halogène, a groupe alkyle ayant 1 à 18 atomes de carbone, a groupe alkyloxy ayant 1 à 18 atomes de carbone, -CO-R² ou 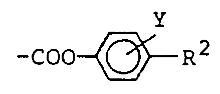 où R² est a groupe alkyle ou alkyloxy ayant 1 à 18 atomes de carbone et Y est tel que défini ci-dessus.

6. La composition selon la Revendication 5, dans laquelle la teneur dudit composé optiquement actif (1) va de 0,1 à 20 % en masse.

7. La composition selon la Revendication 5, présentant une phase smectique C possédant le pouvoir rotatoire.

8. La composition selon la Revendication 5, présentant une phase nématique possédant le pouvoir rotatoire.

9. L'utilisation du composé optiquement actif selon la Revendication 1, sous forme d'un additif pour des cristaux liquides.
